# EUROPÄISCHE PATENTANMELDUNG

(11) **EP 3 738 558 A1**
(43) Veröffentlichungstag der Anmeldung: **18.11.2020**
(21) Anmeldenummer: 19175168.4
(22) Anmeldetag: 17.05.2019
(51) Int. Cl.: A61F 9/007, A61B 90/30, A61B 3/00, G02B 21/00, A61B 17/02, A61B 1/06, A61B 1/07

(54) **LICHTINSTRUMENT ZUR BELEUCHTUNG DES INTRAOKULAREN RAUMES**

(71) Anmelder: Oertli-Instrumente AG, 9442 Berneck (CH)
(72) Erfinder: KEHREN-QUITSDORF, Lisa, 9320 Arbon (CH); KNÜNZ, Lothar, 6830 Rankweil (AT); ZÜRCHER, Michael, 9000 St. Gallen (CH)
(74) Vertreter: Piticco, Lorena

(57) **Zusammenfassung**

Eine Vorrichtung (2) für ein ophthalmologisches Beleuchtungssystem (1) umfassend ein Lichtinstrument (3) zur Beleuchtung des intraokularen Raumes eines menschlichen oder tierischen Auges (33) umfasst ein Gehäuse (4) mit einem proximalen Gehäuseende (5), einem distalen Gehäuseende (6), und eine Öffnung (7) im proximalen Gehäuseende (5). Das Gehäuse (4) begrenzt einen Aufnahmeraum (8), welcher sich ausgehend von der Öffnung (7) im proximalen Gehäuseende (5) entlang einer Längsrichtung (L) in Richtung des distalen Gehäuseendes (6) erstreckt. Der Aufnahmeraum (8) ist zur Aufnahme des Lichtinstruments (3) durch die Öffnung (7) im proximalen Gehäuseende (5) ausgebildet. Das Gehäuse (4) umfasst zumindest im Bereich (10) des distalen Gehäuseendes (6) mindestens ein transluzentes Material.

## Beschreibung

### TECHNISCHES GEBIET

Die vorliegende Erfindung betrifft eine Vorrichtung für ein ophthalmologisches Beleuchtungssystem umfassend ein Lichtinstrument zur Beleuchtung des intraokularen Raumes eines menschlichen oder tierischen Auges gemäss Anspruch 1. Weiter betrifft die vorliegende Erfindung ein ophthalmologisches Beleuchtungssystem umfassend eine solche Vorrichtung gemäss Anspruch 13 sowie ein Verfahren zur Herstellung einer Vorrichtung für ein ophthalmologisches Beleuchtungssystem umfassend ein Lichtinstrument zur Beleuchtung des intraokularen Raumes eines menschlichen oder tierischen Auges gemäss Anspruch 15.

### STAND DER TECHNIK

Durch den physiologischen Aufbau des Auges sind die peripheren Bereiche der Retina (Netzhaut) nicht durch das Operationsmikroskop sichtbar. Auch unter Verwendung von zusätzlicher Weitwinkeloptik ist der Öffnungswinkel der im Operationsmikroskop verwendeten Optik zu klein. Um dennoch in den peripheren Bereichen operieren zu können, muss der entsprechende Bereich im Sichtfeld des Anwenders liegen, wobei der Anwender gleichzeitig mit Instrumenten an genau diesen Bereich gelangen muss. Für diese Operationssituation sind mehrere Möglichkeiten aus dem Stand der Technik bekannt. So gibt es die sogenannte 4-Port-Technik, wobei eine Beleuchtung über ein im Auge fixiertes Lichtinstrument und ein Eindellen respektive Vorbuckeln respektive eine Indentation mittels eines Sklera-Depressors erfolgt. Nachteilig bei dieser Technik sind die Verwendung eines kostenintensiven Lichtinstruments sowie die zusätzliche Verletzung des Patientenauges aufgrund des vierten Zugangs ins Auge. Der Sklera-Depressor ist üblicherweise in der Form eines Stiftes oder in Form eines Fingerhutes ausgeführt, siehe zum Beispiel US 2008/0081952 A1. Darin wird ebenfalls offenbart, dass eine Beleuchtungsquelle wie eine LED in den Sklera-Depressor integriert werden kann um die Beleuchtung im Operationsraum zu verbessern. Eine andere Möglichkeit besteht darin, eine Hilfeleistung durch einen Assistenzarzt zu beanspruchen. Dabei kann einerseits eine Beleuchtung über ein Endo-Lichtinstrument und andererseits ein Eindellen oder Vorbuckeln mithilfe eines unbeleuchteten Hilfsinstruments durch den Assistenzarzt erfolgen. Hierzu muss der Assistenzarzt allerdings über die nötigen Fähigkeiten verfügen. Weiter ist es so, dass auch ein sehr gut eingespieltes Team aus Chirurg und Assistenzarzt nicht dieselbe Geschwindigkeit und Präzision erreicht, wie wenn der Chirurg alle Operationsschritte eigenständig durchführt. Zudem erhöht die Anwesenheit eines Assistenzarztes die Kosten für den Eingriff.

### DARSTELLUNG DER ERFINDUNG

Es ist eine Aufgabe der vorliegenden Erfindung, die Nachteile des Standes der Technik zu überwinden. Insbesondere soll eine Vorrichtung für ein ophthalmologisches Beleuchtungssystem angegeben werden, welche eine sichere und einfache Handhabung und gleichzeitig eine gute Beleuchtung gewährleistet.

Diese Aufgabe wird durch eine Vorrichtung gemäss Anspruch 1 gelöst. So wird eine Vorrichtung für ein ophthalmologisches Beleuchtungssystem umfassend ein Lichtinstrument zur Beleuchtung des intraokularen Raumes eines menschlichen oder tierischen Auges angegeben, wobei die Vorrichtung ein Gehäuse mit einem proximalen Gehäuseende, einem distalen Gehäuseende, und eine Öffnung im proximalen Gehäuseende umfasst. Das Gehäuse begrenzt einen Aufnahmeraum, welcher sich ausgehend von der Öffnung im proximalen Gehäuseende entlang einer Längsrichtung in Richtung des distalen Gehäuseendes erstreckt. Der Aufnahmeraum ist zur Aufnahme des Lichtinstruments durch die Öffnung im proximalen Gehäuseende ausgebildet. Das Gehäuse umfasst zumindest im Bereich des distalen Gehäuseendes mindestens ein transluzentes Material.

Ein transluzentes Material ist ein Material, welches partiell lichtdurchlässig ist. Oder anders gesagt lässt das transluzente Material Licht teilweise durch. Nochmals anders gesagt ist das transluzente Material nicht transparent, sondern teilweise transparent. Indem das Gehäuse zumindest im Bereich des distalen Gehäuseendes mindestens ein transluzentes Material umfasst wird eine sehr gleichmässige Lichtverteilung des von dem Lichtinstrument emittierten Lichts erreicht. Dies ist auf die intrinsischen Eigenschaften von transluzentem Material zurückzuführen, welches eine starke Streuung und Reflexion von auftreffendem Licht bewirkt. Lichtreflexe, welche den Anwender während der Nutzung stören, werden dadurch vermieden. Dadurch, dass das Lichtinstrument in der Vorrichtung aufnehmbar ist, muss der Anwender nicht mehrere Instrumente bedienen. Die Vorrichtung erlaubt somit eine sichere und einfache Handhabung und gewährleistet gleichzeitig eine gute Beleuchtung.

Vorzugsweise besteht die Vorrichtung im Bereich des distalen Gehäuseendes aus mindestens einem transluzenten Material. Weiter ist es bevorzugt, dass das gesamte Gehäuse mindestens ein transluzentes Material umfasst, und insbesondere bevorzugt aus mindestens einem transluzenten Material besteht.

Das transluzente Material ist vorzugsweise ein transluzenter Kunststoff, insbesondere ein transluzenter technischer Kunststoff, bevorzugt ein transluzenter teilkristalliner Kunststoff, am bevorzugtesten ein Polyoxymethylen Copolymer. Zusätzlich oder alternativ dazu ist es ebenfalls denkbar, das transluzente Material in Form von einem Kunststoff und/oder einem Silikon mit Partikeln, vorzugsweise Metalloxide wie Titandioxid (TiO₂), bereitzustellen. Die Partikel sind vorzugsweise dazu ausgebildet, das aus dem Lichtinstrument austretende Licht zu streuen. Diese Partikel können somit auch als Streupartikel bezeichnet werden. Wird das transluzente Material durch einen Kunststoff mit Streupartikeln bereitgestellt, so kann es sich beim Kunststoff auch um einen transparenten Kunststoff handeln, wobei die Transluzenz oder Teiltransparenz des zumindest distalen Gehäuseendes durch die Streuung des Lichts an den Streupartikeln bereitgestellt wird. Denkbare transparente Kunststoffe sind Thermoplasten, technische Kunststoffe oder teilkristalline Kunststoffe wie sie dem Fachmann bekannt sind. Dabei ist es denkbar, dass die Vorrichtung zumindest im Bereich des distalen Gehäuseendes, entsprechend aber auch in anderen Bereichen oder vollständig, aus nur einem einzigen transluzenten Material oder aber aus einer Mischung von zwei oder mehreren transluzenten Materialien besteht.

Zusätzlich oder alternativ dazu ist es bevorzugt, dass das transluzente Material eine Absorptionskonstante von etwa 10^(-3) im sichtbaren Wellenlängenbereich aufweist.

Denkbar ist, dass die Absorptionskonstante zwischen etwa 1·10^(-3) und 9·10^(-3) im sichtbaren Wellenlängenbereich liegt. Zusätzlich oder alternativ dazu ist es allerdings auch denkbar, dass das distale Gehäuseende im sichtbaren Wellenlängenbereich eine Transmission von mindestens 60 %, vorzugsweise von mindestens 70 %, insbesondere bevorzugt von mindestens 80 % aufweist. Hierzu gilt es zu verstehen, dass mit dem Begriff "Transmission" die gesamte Transmission gemeint ist, deren Hauptkomponente vom transluzenten Material herführt, wobei allerdings noch zusätzliche Nebenkomponenten wie die geometrische Ausgestaltung des distalen Gehäuseendes eine Rolle spielen. Dieser Aspekt wird später eingehender erläutert.

Vorzugsweise ist die Vorrichtung zur Indentation des Augengewebes, insbesondere der Sklera, ausgebildet. Eine Indentation, auch Eindellen oder Vorbuckeln genannt, ist ein räumliches Verschieben des Augengewebes mit einem Hilfsinstrument. Durch diese Verschiebung gelangt Gewebe von Interesse in das Sichtfeld des Anwenders, also der Chirurgin oder des Chirurgen. Damit kann diese / dieser die gewünschten Eingriffe in dem so verschobenen Bereich vornehmen. Vorzugsweise handelt es sich bei der Vorrichtung also sowohl um ein Beleuchtungsinstrument als auch um einen sogenannten Sklera-Depressor (englisch: sclera depressor), welcher zur Indentation der Sklera ausgebildet ist. Oder anders gesagt ist die Vorrichtung bevorzugt ein beleuchteter Sklera-Depressor. Dies bringt den Vorteil, dass Licht auch ohne Trokar und somit ohne einen Zugang oder Port im Auge ins hintere Augensegment gelangen kann. Weitere Vorteile liegen in den geringen Kosten, da ein Sklera-Depressor ohne zusätzliches Beleuchtungsinstrument verwendet werden kann, und da der Anwender keinen Assistenzarzt benötigt. Wird das transluzente Material durch einen teilkristallinen Kunststoff wie Polyoxymethylen Copolymer bereitgestellt, so zeichnet sich die Vorrichtung zudem durch gute Gleiteigenschaften aufgrund eines niedrigen Reibungskoeffizienten aus.

Das Gehäuse umfasst vorzugsweise mindestens einen ersten Bereich und einen sich daran anschliessenden zweiten Bereich, wobei das distale Gehäuseende im ersten Bereich angeordnet ist, wobei ein Aussendurchmesser des ersten Bereichs grösser als ein Aussendurchmesser des zweiten Bereichs ist, und wobei ein Verhältnis zwischen dem Aussendurchmesser des ersten Bereichs und dem Aussendurchmesser des zweiten Bereichs insbesondere mehr als 1 beträgt. Zusätzlich oder alternativ dazu ist es bevorzugt, wenn das Verhältnis zwischen dem Aussendurchmesser des ersten Bereichs und dem Aussendurchmesser des zweiten Bereichs zwischen 1.1 und 2.0, vorzugsweise zwischen 1.3 und 1.7, und insbesondere bevorzugt etwa 1.5 beträgt. Ein denkbarer Aussendurchmesser des ersten Bereichs ist vorzugsweise zwischen 2 Millimeter und 8 Millimeter, insbesondere etwa 6 Millimeter. Ein denkbarer Aussendurchmesser des zweiten Durchmessers ist vorzugsweise zwischen 0.5 Millimeter und 6 Millimeter, insbesondere zwischen etwa 1 Millimeter und 4 Millimeter.

Das heisst, der erste Bereich, oder auch distale Bereich, des Gehäuses weist einen grösseren Aussendurchmesser auf als der sich daran anschliessende zweite Bereich beziehungsweise der zweite Bereich ist bezüglich dem ersten Bereich dünner ausgebildet. Dadurch wird gewährleistet, dass genügend Platz zwischen den Augenaussenflächen wie dem Gewebe und den Augenhöhlen wie den Schädelknochen, der Muskulatur, der Augenwand, dem Fettgewebe, etc. und allfälligen weiteren Instrumenten wie zum Beispiel einem Lidsperrer vorhanden ist, was der Vorrichtung Bewegungsfreiheit verleiht und gestattet, dass sich die Vorrichtung während dem Eindellen einfach auf der Augenoberfläche bewegt.

Vorzugsweise ist das distale Gehäuseende im Wesentlichen kalottenförmig ausgebildet oder weist die Form einer im Wesentlichen zumindestens einseitig abgeflachten Kugel auf. Zusätzlich oder alternativ dazu ist es bevorzugt, wenn das distale Gehäuseende bezüglich der Längsrichtung einen Öffnungswinkel für austretendes Licht des Lichtinstruments von grösser als 100°, vorzugsweise von etwa 110° gemäss EN-ISO 15752:2010 definiert. Zusätzlich oder alternativ dazu ist es bevorzugt, wenn das Gehäuse im Bereich des distalen Gehäuseendes einen kanonischen Raumwinkel definiert, welcher 2·π (Pi) Steradiant beträgt, wobei dieser Raumwinkel einen Mantel eines geraden Kreiskegels mit einem halben, ebenen Öffnungswinkel von 90° bildet, so dass innerhalb dieses Raumwinkels eine Strahlungsstärke des aus dem Bereich des distalen Gehäuseendes austretenden Lichts des Lichtinstruments mindestens 30 %, vorzugsweise mindestens 60 %, am bevorzugtesten mindestens 90 % der Strahlungsstärke des Lichts des Lichtinstruments innerhalb dieses Raumwinkels beträgt. Diese Ausgestaltung führt zu einer geringen Leistungsabnahme des Lichts über einen grossen Winkelbereich. Wie eingangs bereits erwähnt wurde, zeichnet sich die Vorrichtung durch einen sehr geringen Lichtverlust aus, was einerseits durch das transluzente Material selbst ermöglicht wird. Andererseits führt die Kugel- bzw. Kalottenform zu einer Reflexion des Lichts an einen anderen räumlichen Ort. Das heisst, die Kombination aus transluzentem Material und einer Kugel- oder Kalottenform bewirken eine Homogenisierung des austretenden Lichts, wobei ein Verlust an Strahlungsstärke des austretenden Lichts im Wesentlichen einzig aufgrund der (geringen) Absorption des transluzenten Materials erfolgt. Die einseitig abgeflachte Kugel bewirkt, dass eine Breite der Kugel, oder anders gesagt die Dicke der Kugel, vergrössert werden kann. Dies ergibt den Vorteil, dass flachere Winkel zwischen dem Augengewebe und der Kugeloberfläche des distalen Gehäuseendes gebildet werden, was wiederum zu einem geringeren Verfahrwiderstand und einer entsprechend geringeren Faltenbildung führt. Zudem kann ein grösserer Bereich des Augengewebes eingedellt werden, wodurch der Eingriff schneller durchgeführt und weiter entfernte Bereiche des Augengewebes erreicht werden können.

Eine kalottenförmige oder kugelförmige Ausgestaltung bringt den Vorteil, dass der distale Endbereich verrundete Kanten und dabei keine Schlitze oder andere Unebenheiten umfasst. Das Risiko einer Verletzung der Bindehaut sowie eine Faltenbildung werden dadurch verringert oder sogar verhindert. Weiter führen diese Ausgestaltungen zu einer im Wesentlichen symmetrischen Abstrahlung des Lichts von der Vorrichtung. Der Anwender kann sich daher auf das Eindellen beziehungsweise die eigentlichen Schritte der Operation konzentrieren und muss sich nicht mit der Ausrichtung der Vorrichtung beschäftigen. Zudem führen diese Ausgestaltungen zu einem geringeren Verfahrwiderstand.

Vorzugsweise umfasst das Gehäuse einen dritten Bereich, wobei das proximale Gehäuseende im dritten Bereich angeordnet ist, und wobei der dritte Bereich ausgehend vom proximalen Gehäuseende in Richtung des distalen Gehäuseendes gesehen zumindest teilweise in Richtung der Längsrichtung nach innen zulaufend ausgebildet ist.

Das heisst es ist bevorzugt, dass das Gehäuse einen ersten oder distalen Bereich mit einer Kalotten- oder zumindest einseitig abgeflachten Kugelform, einen sich daran anschliessenden zweiten oder mittleren Bereich mit einer gegenüber dem ersten oder distalen Bereich geringeren Aussendurchmesser, sowie einen sich an diesen zweiten oder mittleren Bereich anschliessenden konisch zulaufenden dritten oder proximalen Bereich umfasst. Vorzugsweise ist der zweite Bereich im Wesentlichen zylindrisch ausgebildet und ist bezüglich der Längsrichtung länger ausgebildet als der erste und der dritte Bereich. Der zweite Bereich kann als länglicher Schaft gesehen werden. Der Aufnahmeraum erstreckt sich vorzugsweise vollständig durch den dritten Bereich sowie den zweiten Bereich hindurch und zumindest teilweise in den ersten Bereich hinein.

Es ist bevorzugt, dass eine lichte Weite des Aufnahmeraums ausgehend vom proximalen Gehäuseende in Richtung des distalen Gehäuseendes gesehen vorzugsweise im Wesentlichen kontinuierlich abnimmt. Durch diesen ausgehend vom distalen Gehäuseende in Richtung des proximalen Gehäuseendes gesehenen grösser werdenden Innendurchmesser des Gehäuses steigt zudem dessen Biegefestigkeit. Weiter ist ein Verhältnis zwischen dem Aussendurchmesser des zweiten oder mittleren Bereichs und einem Innendurchmesser dieses zweiten oder mittleren Bereichs vorzugsweise grösser als 1, insbesondere grösser als 1.1.

Vorzugsweise hat das Gehäuse im Bereich des distalen Gehäuseendes bezüglich einer senkrecht zur Längsrichtung verlaufenden Querrichtung eine Wanddicke von etwa 0.5 mm bis 3 mm, insbesondere von etwa 1.5 mm. Zusätzlich oder alternativ dazu hat das Gehäuse im Bereich des distalen Gehäuseendes entlang der Längsrichtung gesehen eine Wanddicke von etwa 0.5 mm bis 3 mm, insbesondere von etwa 1 mm.

Das heisst, das Gehäuse ist zumindest im Bereich des distalen Gehäuseendes, also im ersten respektive distalen Bereich, mit einer geringen Materialstärke ausgebildet. Die geringe Materialstärke reduziert Lichtverluste durch Absorption und führt zu einer Erhöhung der von der Vorrichtung abgestrahlten Lichtleistung.

Das Gehäuse weist vorzugsweise auf einer Aussenseite zumindest im Bereich des distalen Gehäuseendes eine Rauheit von etwa 0.2 bis 2.2 Ra gemäss EN ISO 1302, vorzugsweise von etwa 0.4 bis 2 Ra gemäss EN ISO 1302, insbesondere bevorzugt von etwa 0.6 bis 0.8 Ra gemäss EN ISO 1302 auf. Vorzugsweise weisen der zweite Bereich und/oder der dritte Bereich des Gehäuses auf ihrer Aussenseite jeweils dieselben Rauheiten wie der erste Bereich bzw. der Bereich des distalen Gehäuseendes auf. Alternativ ist es auch denkbar, diese Bereiche mit unterschiedlichen Rauheiten bereitzustellen.

Vorzugsweise ist die Vorrichtung mit dem Lichtinstrument lösbar verbindbar, wobei das Gehäuse insbesondere zur Ausbildung einer formschlüssigen und/oder kraftschlüssigen Verbindung mit dem Lichtinstrument ausgebildet ist. Bei der Vorrichtung kann es sich um einen Einwegartikel handeln, welcher nach dem Gebrauch vom Lichtinstrument entfernt und entsorgt wird. Im Falle eines neuen Eingriffst kann eine neue Vorrichtung mit dem Lichtinstrument verbunden werden. Dadurch ist ein Mehrfachgebrauch des Lichtinstruments möglich.

Weiter ist es denkbar, die Dimension des dritten beziehungsweise proximalen Bereichs des Gehäuses derart zu wählen, dass diese im Wesentlichen der Dimension des Lichtinstruments im Bereich der Verbindung mit der Vorrichtung entspricht. Im Wesentlichen gleiche Dimensionen heisst in diesem Zusammenhang, dass der dritte Bereich des Gehäuses einen Innendurchmesser definiert, welcher leicht grösser als ein Aussendurchmesser des Lichtinstruments im Bereich der Verbindung mit der Vorrichtung ist. Leicht grösser wiederum heisst, dass der zu verbindende Bereich des Lichtinstruments im Wesentlichen spielfrei im proximalen beziehungsweise dritten Bereich des Gehäuses aufnehmbar ist. Diese Art von Ausgestaltung erlaubt eine sichere und gleichzeitig einfache Verbindung, wobei die Vorrichtung beispielsweise auf das Lichtinstrument aufgesteckt wird.

Vorzugsweise ist die Vorrichtung mit Ausnahme der Öffnung im proximalen Gehäuseende vollständig geschlossen ausgebildet. Oder anders gesagt umgibt das Gehäuse das darin aufgenommene Lichtinstrument vollständig, wodurch das Lichtinstrument von einer Kontamination geschützt wird. Dies erlaubt somit auch eine einfache und sichere Mehrfachverwendung des Lichtinstruments. Das heisst, die Vorrichtung kann während eines Eingriffs beim selben Patienten zeitweise von Lichtinstrument entfernt und zu einem späteren Zeitpunkt wieder am Lichtinstrument angebracht werden. Die Vorrichtung kann also während demselben Eingriff mehrmals verwendet werden.

Das Gehäuse verfügt vorzugsweise im Bereich des proximalen Gehäuseendes auf einer dem Aufnahmeraum zugewandten Innenseite über mindestens eine Verstärkungsrippe. Insbesondere bevorzugt sind mehrere Verstärkungsrippen vorhanden, welche sich jeweils parallel zur Längsrichtung erstrecken. Die Verstärkungsrippen erhöhen die Biegesteifigkeit der Vorrichtung. Weiter ist es von Vorteil, wenn zwischen den Verstärkungsrippen jeweils ein Zwischenraum ausgebildet ist, in welchen entsprechend ausgebildete Vorsprünge am Lichtinstrument aufnehmbar sind. In diesem Fall ermöglichen die Verstärkungsrippen eine formschlüssige Verdrehsicherheit bezüglich einer ungewollten Verdrehung der Vorrichtung relativ zum Lichtinstrument. Dadurch kann ein unbeabsichtigtes Lösen der Vorrichtung vom Lichtinstrument verhindert werden.

In einem weiteren Aspekt wird ein ophthalmologisches Beleuchtungssystem umfassend eine Vorrichtung wie oben beschrieben und ein Lichtinstrument angegeben, wobei das Lichtinstrument vorzugsweise einen Lichtleiter zum Leiten von Licht, beispielsweise eines Lichtstrahls umfasst. Beim Lichtleiter handelt es sich bevorzugt um eine Faser.

Es gilt zu verstehen, dass sämtliche Aussagen, welche oben für die Vorrichtung gemacht wurden, analog für die Vorrichtung beim ophthalmologischen Beleuchtungssystem gelten.

Der Lichtleiter ist derart im Aufnahmeraum der Vorrichtung gelagert, dass ein Verhältnis zwischen i) einem Abstand zwischen einem distalen Ende des Lichtleiters und einem Zentrum des distalen Gehäuseendes, insbesondere dem Zentrum des im Wesentlichen kalottenförmigen distalen Gehäuseendes oder des distalen Gehäuseendes in Form der im Wesentlichen zumindest einseitig abgeflachten Kugel und ii) dem Aussendurchmesser des ersten Bereichs des Gehäuses, insbesondere der Aussendurchmesser im Bereich des Zentrums des im Wesentlichen kalottenförmigen distalen Gehäuseendes oder des distalen Gehäuseendes in Form der im Wesentlichen zumindest einseitig abgeflachten Kugel kleiner als 1.5 ist. Zusätzlich oder alternativ dazu ist das distale Ende des Lichtleiters vorzugsweise im Zentrum des im Wesentlichen kalottenförmigen distalen Gehäuseendes oder des distalen Gehäuseendes in Form der im Wesentlichen zumindest einseitig abgeflachten Kugel angeordnet. Als Zentrum wird hier die Stelle innerhalb des Aufnahmeraums verstanden, an welcher das distale Gehäuseende bezüglich einer senkrecht zur Längsrichtung verlaufenden Querrichtung über seine maximale Ausdehnung beziehungsweise den grössten Aussendurchmesser verfügt.

Diese Ausgestaltung beziehungsweise Anordnung in Kombination mit dem homogen streuenden Material führt dazu, dass Lichtreflexe aufgeweitet werden und sich die Leuchtdichte verringert. Somit lassen sich Reflexe, die den Anwender während der Nutzung stören können, vermeiden. Indem nämlich ein Abstand zwischen dem distalen Ende des Lichtleiters und dem distalen Gehäuseende besteht, wird der distale oder erste Bereich der Vorrichtung nicht komplett vom Augen-Gewebe umgeben. Entsprechend wird Licht nicht oder nur teilweise vom Gewebe absorbiert.

In einem weiteren Aspekt wird ein Verfahren zur Herstellung einer Vorrichtung für ein ophthalmologisches Beleuchtungssystem umfassend ein Lichtinstrument, insbesondere einer Vorrichtung wie oben beschrieben, zur Beleuchtung des intraokularen Raumes eines menschlichen oder tierischen Auges angegeben, wobei das Verfahren den Schritt umfasst von:
Spritzgiessen eines Gehäuses mit einem proximalen Gehäuseende, einem distalen Gehäuseende, und einer Öffnung im proximalen Gehäuseende unter Verwendung eines Spritzgusswerkzeuges, wobei ein Aufnahmeraum im Gehäuse gebildet wird, welcher sich ausgehend von der Öffnung im proximalen Gehäuseende entlang einer Längsrichtung in Richtung des distalen Gehäuseendes erstreckt, und wobei zumindest für den Bereich des distalen Gehäuseendes mindestens ein transluzentes Material verwendet wird.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Fig. 1: zeigt eine teilweise Schnittansicht eines ophthalmologischen Beleuchtungssystems umfassend ein Lichtinstrument und eine Vorrichtung gemäss einer ersten Ausführungsform während einer chirurgischen Anwendung;
- Fig. 2: zeigt eine perspektivische Ansicht des ophthalmologischen Beleuchtungssystems gemäss Figur 1, wobei die Vorrichtung vom Lichtinstrument getrennt ist;
- Fig. 3: zeigt eine perspektivische Ansicht des ophthalmologischen Beleuchtungssystems gemäss Figur 1, wobei die Vorrichtung mit dem Lichtinstrument verbunden ist;
- Fig. 4: zeigt eine teilweise Schnittansicht durch die Vorrichtung gemäss Figur 1;
- Fig. 5a: zeigt einen zentralen Längsschnitt durch die Vorrichtung und einen Teil des Lichtinstruments gemäss Figur 1 mit dem Lichtinstrument an einer ersten Position;
- Fig. 5b: zeigt einen zentralen Längsschnitt durch die Vorrichtung und einen Teil des Lichtinstruments gemäss Figur 1 mit dem Lichtinstrument an einer zweiten Position;
- Fig. 6: zeigt einen weiteren zentralen Längsschnitt durch die Vorrichtung gemäss Figur 1;
- Fig. 7: zeigt einen weiteren zentralen Längsschnitt durch einen Teil der Vorrichtung gemäss Figur 1;
- Fig. 8: zeigt einen weiteren zentralen Längsschnitt durch einen Teil einer Vorrichtung gemäss einer zweiten Ausführungsform;
- Fig. 9: zeigt eine perspektivische Ansicht einer Vorrichtung gemäss einer dritten Ausführungsform;
- Fig. 10a: zeigt eine teilweise perspektivische Ansicht des Lichtinstruments gemäss der ersten Ausführungsform;
- Fig. 10b: zeigt eine Seitenansicht des Lichtinstruments gemäss Figur 10a;
- Fig. 10c: zeigt eine Teilschnittansicht des Lichtinstruments gemäss Figur 10a;
- Fig. 10d: zeigt eine weitere perspektivische Ansicht des Lichtinstruments gemäss Figur 10a;
- Fig. 11a: zeigt eine teilweise perspektivische Ansicht des Lichtinstruments gemäss einer zweiten Ausführungsform;
- Fig. 11b: zeigt eine Seitenansicht des Lichtinstruments gemäss Figur 11a;
- Fig. 11c: zeigt eine Teilschnittansicht durch das Lichtinstrument gemäss Figur 11a;
- Fig. 12: zeigt eine schematische Teilansicht der Vorrichtung gemäss der ersten Ausführung und deren Abstrahlcharakteristik verdeutlicht durch einen Raumwinkel und dessen halben, ebenen Öffnungswinkel;
- Fig. 13a: zeigt Messungen der normierten Strahlungsstärke in [%] von einem Lichtinstrument gemäss der ersten Ausführungsform in einer ersten Ausrichtung bezüglich der Öffnungswinkel in Polarkoordinaten;
- Fig. 13b: zeigt Messungen der normierten Strahlungsstärke in [%] vom Lichtinstrument gemäss Figur 13a in einer bezüglich der in Figur 13a gezeigten Ausrichtung um 90 Grad verdrehten zweiten Ausrichtung bezüglich der Öffnungswinkel in Polarkoordinaten;
- Fig. 14: zeigt Messungen der normierten Strahlungsstärke in [%] von einem Lichtinstrument gemäss der zweiten Ausführungsform bezüglich der Öffnungswinkel in Polarkoordinaten;
- Fig. 15a: zeigt Messungen der normierten Strahlungsstärke in [%] von einer Vorrichtung gemäss der ersten Ausführungsform mit einer ersten Wandstärke mit unterschiedlichen Typen von Lichtinstrumenten gemäss der ersten und zweiten Ausführungsform bezüglich der Öffnungswinkel in Polarkoordinaten;
- Fig. 15b: zeigt die Messungen der normierten Strahlungsstärke in [%] bezüglich der Öffnungswinkel der Vorrichtung und der unterschiedlichen Typen von Lichtinstrumenten gemäss Figur 15a in kartesischen Koordinaten;
- Fig. 16a: zeigt Messungen der normierten Strahlungsstärke in [%] von einer Vorrichtung gemäss der ersten Ausführungsform mit einer zweiten Wandstärke mit unterschiedlichen Typen von Lichtinstrumenten gemäss der ersten und zweiten Ausführungsform bezüglich der Öffnungswinkel in Polarkoordinaten;
- Fig. 16b: zeigt die Messungen der normierten Strahlungsstärke in [%] bezüglich der Öffnungswinkel der Vorrichtung und der unterschiedlichen Typen von Lichtinstrumenten gemäss Figur 16a in kartesischen Koordinaten.

### BESCHREIBUNG BEVORZUGTER AUSFÜHRUNGSFORMEN

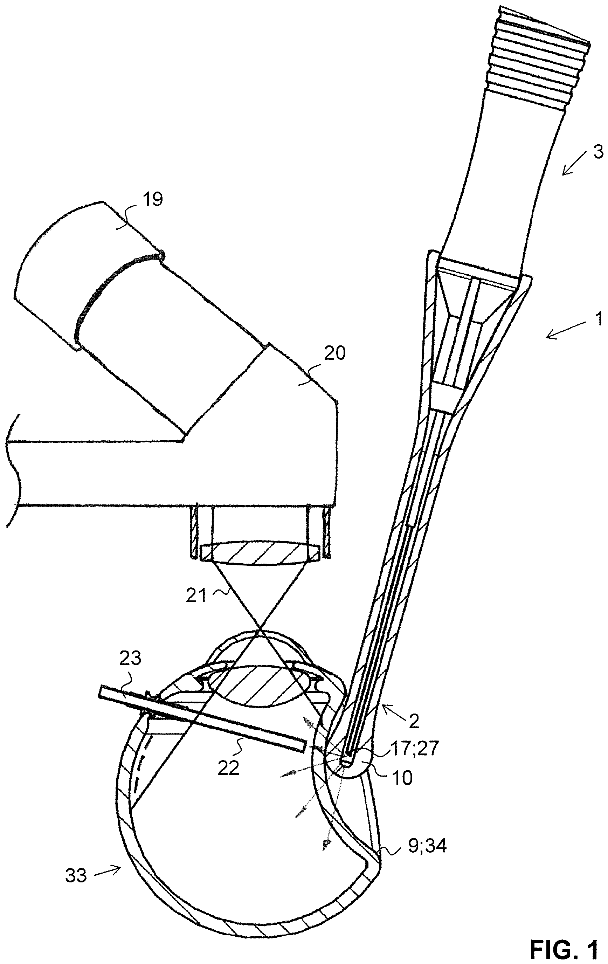

Im Zusammenhang mit den Figuren 1 bis 16 werden verschiedene Aspekte eines ophthalmologisches Beleuchtungssystems 1 umfassend ein Lichtinstrument 3 sowie eine Vorrichtung 2 zur Beleuchtung des intraokularen Raumes eines menschlichen oder tierischen Auges 33 diskutiert.

Wie insbesondere aus der Figur 1 hervorgeht, dient die Vorrichtung 2 einer Doppelfunktion. Einerseits nämlich ist die Vorrichtung 2 zur Indentation des Augengewebes 34, insbesondere der Sklera 9 ausgebildet. Das heisst, bei der Vorrichtung 2 handelt es sich um einen sogenannten Sklera-Depressor oder Eindeller, mittels welchem das Augengewebe 34 räumlich verschoben werden kann. Durch diese Verschiebung gelangt das Gewebe von Interesse in das Sichtfeld des Anwenders, in der Regel des Chirurgen, so dass dieser in diesem Bereich einen gewünschten Eingriff vornehmen kann. Mit anderen Worten gestattet die Vorrichtung 2 die Sichtbarmachung peripherer Bereiche des Auges wie der Retina und des Vitreous, um in diesen Bereichen chirurgische Eingriffe durchführen zu können. Andererseits dient die Vorrichtung 2 auch einer Beleuchtung. So wird die Vorrichtung mit dem Lichtinstrument 3 des Beleuchtungssystems 1 verbunden, wobei Licht vom Lichtinstrument 3 über die Vorrichtung 2 ins Augeninnere abstrahlt. Die Vorrichtung 2 kann somit als beleuchteter Eindeller oder als beleuchteter Sklera-Depressor bezeichnet werden, welcher eine transsklerale Illumination der peripher gelegenen Bereiche des Bulbus (Augapfel) ermöglicht. Wie später noch ausführlicher erläutert wird, tritt das Licht dabei am distalen Ende 17 des Lichtinstruments 3 aus und transmittiert durch einen distalen Bereich 10 des Sklera-Depressors 2 durch das eingedellte Augengewebe 34 ins Augeninnere. Wie in Figur 1 angedeutet, kann der Anwender die Operation dabei durch das Okular 19 eines Operationsmikroskops 20 beobachten, wobei sein Sichtfeld in Figur 1 durch den Strahlengang 21 dargestellt ist. Bereiche, die nicht im Sichtfeld des Anwenders liegen, werden durch die räumliche Verschiebung mittels des erfindungsgemässen Sklera-Depressors 2 in das Sichtfeld gedrückt. Da die räumliche Verschiebung und die Beleuchtung mit derselben Vorrichtung 2 und mit nur einer Hand erfolgt, bleibt dem Anwender eine freie Hand, mit welcher er andere Instrumente wie z.B. ein Vitrektom 22 für die Glaskörperentfernung, welcher durch einen Trokar 23 ins Augeninnere geführt wird, bedienen kann.

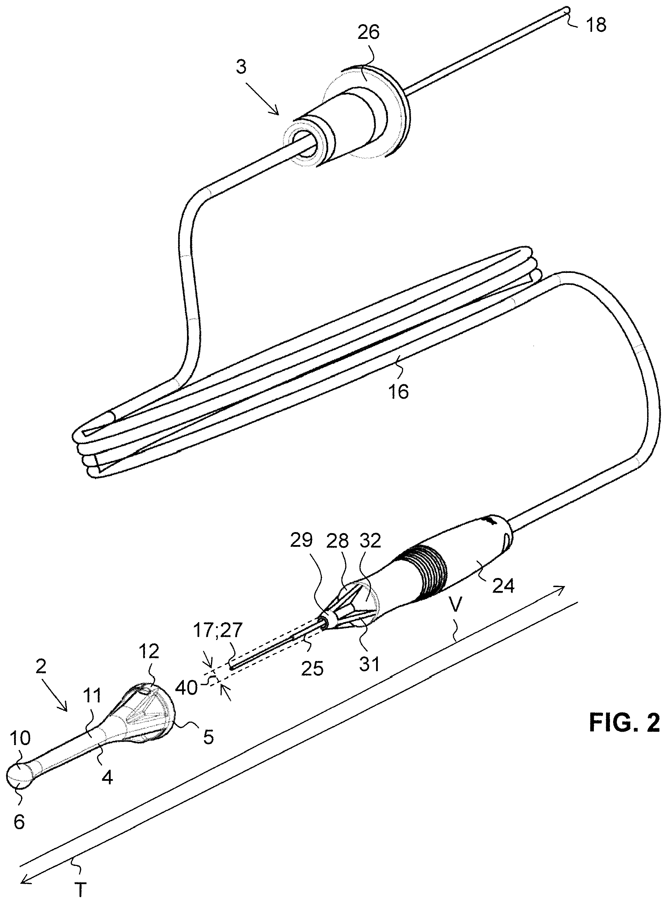

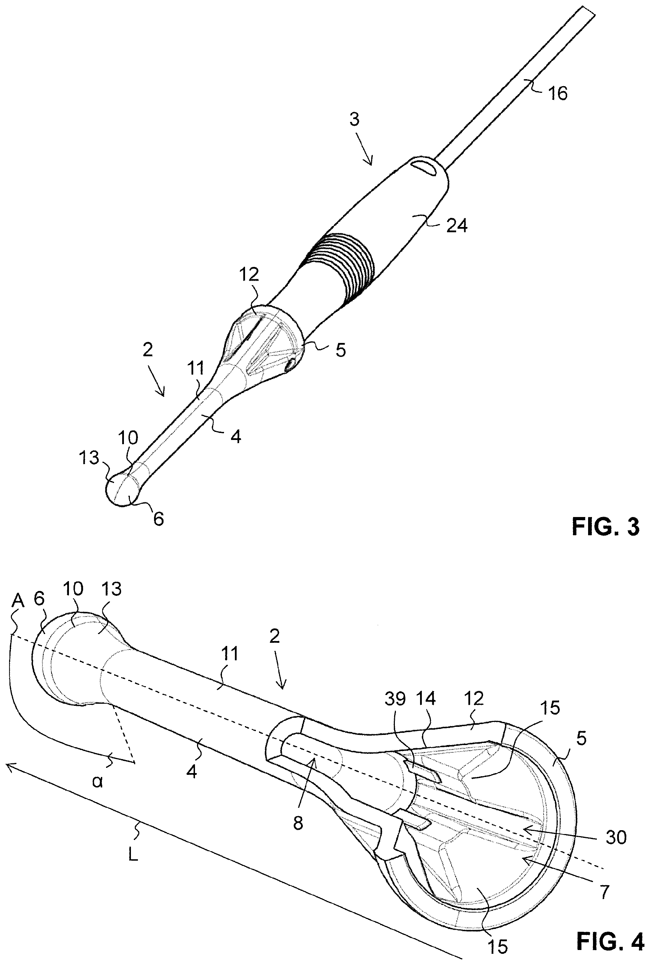

Wie gut in Figur 2 ersichtlich ist, umfass das ophthalmologische Beleuchtungssystem 1 nebst dem Sklera-Depressor 2 und dem Lichtinstrument 3 eine nicht dargestellte Lichtquelle mit einer Buchse. Das Lichtinstrument 3 wiederum umfasst in der gezeigten Ausführungsform einen Handgriff 24, einen Lichtleiter 16, eine Kanüle 25 und einen Stecker 26. Der Lichtleiter 16 ist hier in Form einer Faser zur Verfügung gestellt und weist ein proximales Ende 18 zum Einkoppeln von Licht von der Lichtquelle sowie ein distales Ende 17 zum Emittieren des eingekoppelten Lichts auf. Der Lichtleiter 16 steht mit dem Stecker 26 des Lichtinstruments 3 in fester Verbindung und über die Lichtquelle wird Licht in den Lichtleiter 16 eingekoppelt. Weiter ist die Kanüle 25 am Handgriff 24 befestigt und der Lichtleiter 16 steht mit dem Handgriff 24 und der Kanüle 25 in Verbindung. Der Lichtleiter 16 erstreckt sich dabei durch den Handgriff 24 und die Kanüle 25 bis hin zum distalen Ende 27 der Kanüle 25. Das distale Ende 17 des Lichtleiters 16 befindet sich somit im Bereich des distalen Endes 27 der Kanüle 25, wobei Licht vom Lichtleiter 16 ausgekoppelt werden kann. Insbesondere aus den Figuren 3 bis 6 geht hervor, dass der Sklera-Depressor 2 ein Gehäuse 4 mit einem proximalen Gehäuseende 5, einem distalen Gehäuseende 6, und eine Öffnung 7 im proximalen Gehäuseende 5 umfasst. Das Gehäuse 4 begrenzt einen Aufnahmeraum 8, welcher sich ausgehend von der Öffnung 7 im proximalen Gehäuseende 5 entlang einer Längsrichtung L in Richtung des distalen Gehäuseendes 6 erstreckt. Das Lichtinstrument 3, genau gesagt die Kanüle 25 und der darin gelagerte Lichtleiter 16, werden durch die Öffnung 7 im proximalen Gehäuseende 5 in den Aufnahmeraum 8 des Sklera-Depressors 2 aufgenommen. Im aufgenommenen Zustand kommt das distale Ende 27 der Kanüle 25 und folglich das distale Ende 17 des Lichtleiters 16 im Bereich des distalen Gehäuseendes 6 zu liegen. Dadurch, dass das Gehäuse 4 zumindest im Bereich des distalen Gehäuseendes 6 mindestens ein transluzentes Material umfasst, wird Licht ausgehend vom distalen Ende 17 des Lichtleiters 16 über das distale Ende 27 der Kanüle 25 und durch das distale Gehäuseende 6 abgestrahlt.

Der Sklera-Depressor 2 ist lösbar mit dem Lichtinstrument 3, insbesondere mit einem distalen Bereich 28 des Handgriffs 24, verbindbar. Dazu sind der proximale Bereich 12 des Gehäuses 4 und der distale Bereich 28 des Handgriffs 24 gewissermassen komplementär zueinander ausgebildet. Insbesondere ist der proximale Bereich 12 des Gehäuses 4 ausgehend vom proximalen Gehäuseende 5 in Richtung des distalen Gehäuseendes 6 gesehen in Richtung der Längsrichtung L des Sklera-Depressors 2 nach innen konisch zulaufend ausgebildet. Auch der distale Bereich 28 des Handgriffs 24 ist in Richtung des distalen Endes 29 des Handgriffs 24 gesehen nach innen konisch zulaufend ausgebildet, wobei die Dimensionen der konisch zulaufenden Bereiche 12, 28 derart gewählt sind, dass ein Formschluss zwischen den konisch zulaufenden Bereichen 12, 28 ausgebildet wird wenn der distale Bereich 28 des Handgriffs 24 im proximalen Bereich 12 des Sklera-Depressors 2 aufgenommen ist. Zur Verbindung des Handgriffs 24 mit dem Sklera-Depressors 2 kann der Sklera-Depressor 2 entlang einer Verbindungsrichtung V auf den distalen Bereich 28 des Handgriffs 24 aufgesteckt werden. Um den Sklera-Depressor 2 vom Handgriff 24 zu lösen, zieht der Anwender den Sklera-Depressor 2 entlang einer entgegengesetzt zur Verbindungsrichtung V verlaufenden Trennrichtung T vom distalen Bereich 28 des Handgriffs 24 weg. Dank dieser Verbindungsart muss der Sklera-Depressor 2 während eines Eingriffs nicht festgehalten werden.

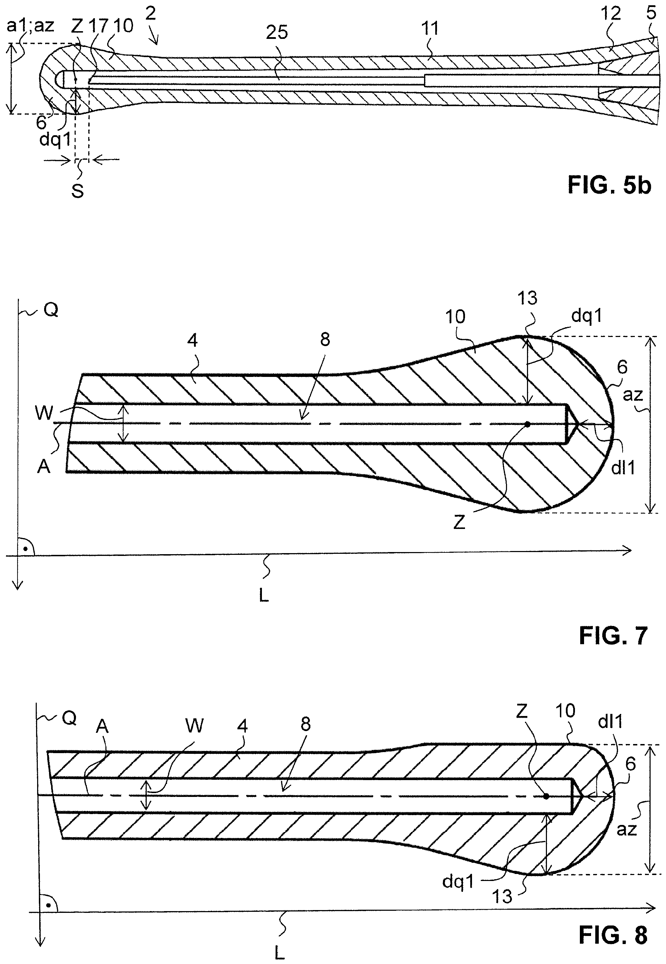

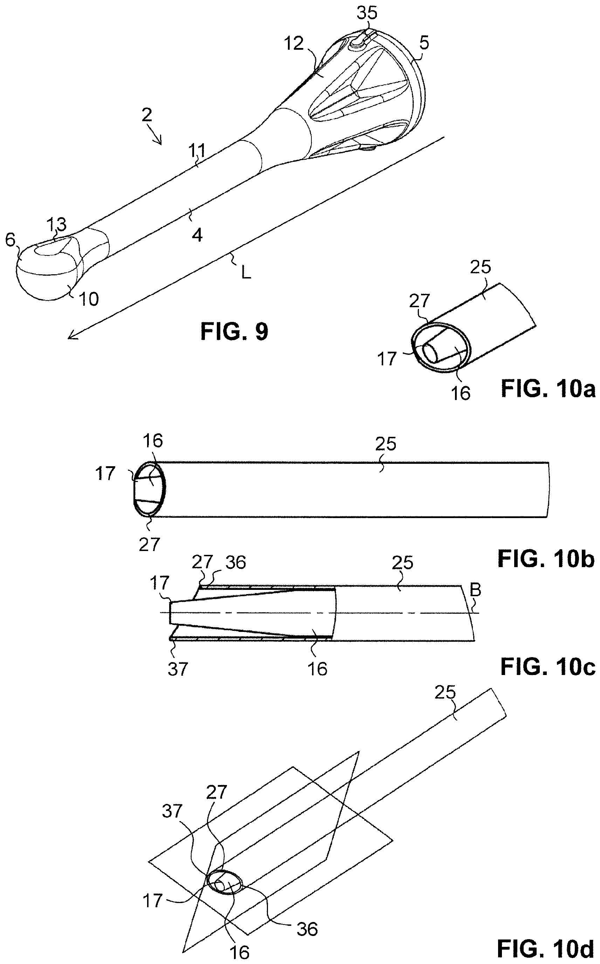

In den Figuren 2 und 4 ist gut ersichtlich, dass das Gehäuse 4 des Sklera-Depressors 2 im Bereich 12 des proximalen Gehäuseendes 5 auf einer dem Aufnahmeraum 8 zugewandten Innenseite 14 über mehrere Verstärkungsrippen 15 verfügt. Die Verstärkungsrippen 15 erstrecken sich dabei von der dem Aufnahmeraum 8 zugewandten Innenseite 14 des Gehäuses 4 parallel zur Längsrichtung L sowie zumindest teilweise in den Aufnahmeraum 8 hinein. Die Verstärkungsrippen 15 dienen einer Erhöhung der Biegefestigkeit des Sklera-Depressors 2. Wie aus Figur 4 hervorgeht, ist zwischen zwei benachbarten Verstärkungsrippen 15 jeweils ein Zwischenraum 30 ausgebildet. In diesen Zwischenräumen 30 werden entsprechende Vorsprünge 31, welche auf einer Aussenseite 32 im distalen Bereich 28 des Handgriffs 24 angeordnet sind, aufgenommen, siehe Figur 2. Dadurch wird ein weiterer Formschluss sowie eine Verdrehsicherung bezüglich einer Verdrehung des Sklera-Depressors 2 relativ zum Handgriff 24 und somit zur Kanüle 25 und dem darin gelagerten Lichtleiter 16 bereitgestellt. Wie weiter aus Figur 4 hervorgeht, umfasst das Gehäuse 4 des Sklera-Depressors 2 im Bereich des proximalen Gehäuseendes 5 auf seiner Innenseite 14 zudem sich entlang der Längsrichtung L erstreckende Rippen 39. Diese Rippen 39 begrenzen einen Innendurchmesser des Gehäuses 4 im Wesentlichen auf einen Aussendurchmesser 40 des Handgriffs 24 (siehe Figur 2), so dass zwischen dem Gehäuse 4 am Ort der Rippen 39 und dem Handgriff 24 am Ort des Gehäuses 4 im Falle eines mit dem Handgriff 24 verbundenen Eindellers 2 ein Kraftschluss ausgebildet wird. In dem in Figur 9 gezeigten Ausführungsbeispiel der Vorrichtung in Form des Sklera-Depressors 2 befindet sich im Bereich 12 des proximalen Gehäuseendes 5 auf einer Aussenseite 13 zudem eine taktile Markierung 35, welche als Erhebung ausgebildet ist und dem Anwender eine intuitive Ausrichtung des Sklera-Depressors 2 ermöglicht. Dies ist insbesondere für einen Sklera-Depressor mit einer einseitig abgeflachten Kugelform von Vorteil, siehe Figur 8.

Vorzugsweise handelt es sich beim Sklera-Depressor 2 um einen Einweggegenstand, welcher nach einem operativen Eingriff entsorgt wird. In den vorliegenden Beispielen ist der Sklera-Depressor 2 mit Ausnahme der Öffnung 7 im proximalen Gehäuseende 5 vollständig geschlossen ausgebildet. Das heisst, bis auf diese Öffnung 7 weist der Sklera-Depressor 2 keine weiteren Öffnungen auf. Die im Aufnahmeraum 8 des Gehäuses 4 aufgenommene Kanüle 25 sowie der darin gelagerte Lichtleiter 16 werden durch das Gehäuse 4 des Sklera-Depressors 2 gegenüber äusseren Einflüssen geschützt. Dadurch wird eine einfache und sichere Mehrfachverwendung des Lichtinstruments 3 während derselben Operation gewährleistet. Beispielsweise kann der Anwender das Lichtinstrument 3 zunächst mit dem erfindungsgemässen Sklera-Depressor 2 gebrauchen, wobei der Lichtleiter 16 und die Kanüle 25 im Aufnahmeraum 8 des Sklera-Depressors 2 aufgenommen sind und vollständig vom Gehäuse 4 des Sklera-Depressors 2 umhüllt werden. Alsdann kann der Anwender die Kanüle 25 und den darin gelagerten Lichtleiter 16 aus dem Aufnahmeraum 8 des Sklera-Depressors 2 herausziehen und diese z.B. in einen Trokar 23 einsetzen, wobei die Kanüle 25 und der darin gelagerte Lichtleiter 16 für eine Endoillumination im Augeninnern verwendet werden.

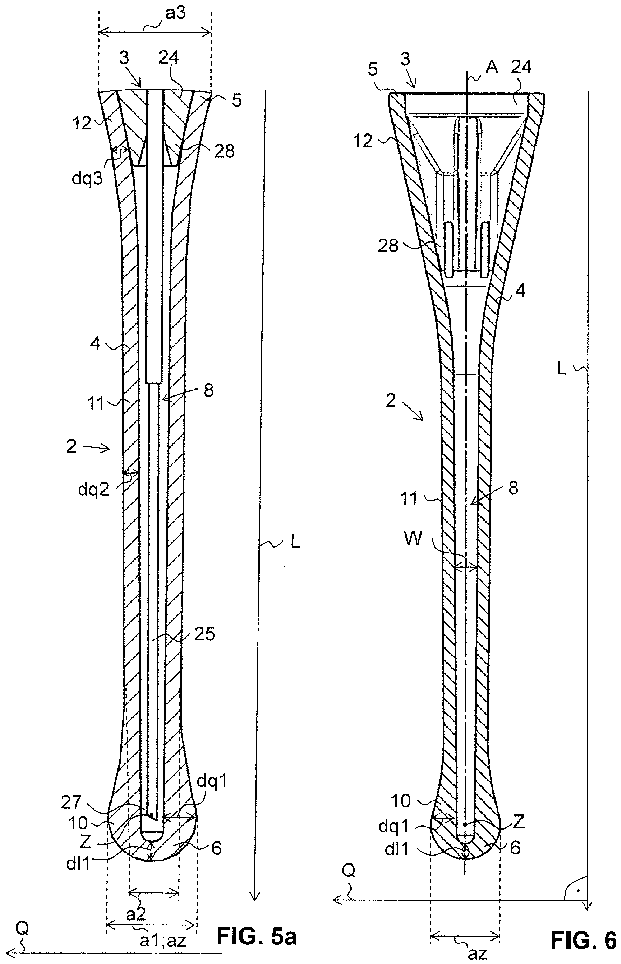

Wie zum Beispiel aus den Figuren 5a, 5b und 6 hervorgeht, weist das Gehäuse 4 des Sklera-Depressors 2 eine längliche Form auf, wobei die Bereiche des proximalen Gehäuseendes 12 und des distalen Gehäuseendes 10 gegenüber dem zwischen diesen Endbereichen 10, 12 ausgebildeten Mittelbereich 11 jeweils einen grösseren Aussendurchmesser a1, a3 aufweisen. Anders gesagt umfasst das Gehäuse 4 einen ersten Bereich 10 und einen sich daran anschliessenden zweiten Bereich 11, wobei das distale Gehäuseende 6 im ersten Bereich 10 angeordnet ist, und wobei ein Aussendurchmesser a1 des ersten Bereichs 10 grösser als ein Aussendurchmesser a2 des zweiten Bereichs 11 ist. Weiter beträgt ein Verhältnis zwischen dem Aussendurchmesser a1 des ersten Bereichs 10 und dem Aussendurchmesser a2 des zweiten Bereichs 11 mehr als 1, hier etwa 1.5. Unterschiedliche Ausführungsformen für den distalen Bereich 10 des Gehäuses 4 sind dabei denkbar. Wie in den Figuren 5 bis 7 und 8 gezeigt wird, kann der distalen Bereich 10 des Gehäuses 4 im Wesentlichen kalottenförmig ausgebildet sein oder die Form einer im Wesentlichen einseitig abgeflachten Kugel aufweisen. Der sich daran anschliessende Mittelbereich bzw. zweite Bereich 11 kann im Wesentlichen zylinderförmig ausgebildet sein und als Schaft bezeichnet werden. Wie früher bereits erwähnt ist der sich an den Mittelbereich bzw. zweiten Bereich 11 anschliessende Bereich 12, also der das proximale Gehäuseende 5 umfassender dritter Bereich 12, ausgehend vom proximalen Gehäuseende 5 in Richtung des distalen Gehäuseendes 6 gesehen konisch nach innen zulaufend ausgebildet. Dabei ist ein Aussendurchmesser a3 des dritten Bereichs 12 an der Stelle des proximalen Gehäuseendes 5 grösser als ein Aussendurchmesser a3 des dritten Bereichs 12 an einer an den zweiten Bereich 11 angrenzenden Stelle. Zudem ist der Aussendurchmesser a3 des dritten Bereichs 12 an der Stelle des proximalen Gehäuseendes 5 grösser als ein Aussendurchmesser a1 des ersten Bereichs 10 an der Stelle des distalen Gehäuseendes 6.

Wie aus den Figuren 5a und 5b hervorgeht, ist das Lichtinstrument 3, hier dessen Kanüle 25 mit dem darin gelagerten Lichtleiter 16, derart im Aufnahmeraum 8 der Vorrichtung 2 aufgenommen, dass das distale Ende 17 des Lichtleiters 16 im Zentrum Z oder in der Nähe des Zentrums Z des kugel- bzw. kalottenförmigen distalen Gehäuseendes 6 zu liegen kommt. Als Zentrum Z wird hier die Stelle innerhalb des Aufnahmeraums 8 verstanden, an welcher das distale Gehäuseende 6 bezüglich einer senkrecht zur Längsrichtung L verlaufenden Querrichtung Q über seine maximale Ausdehnung beziehungsweise den grössten Aussendurchmesser az verfügt. Konkret ist ein Verhältnis zwischen i) einem Abstand S zwischen dem distalen Ende 27 der Kanüle 25 und dem Zentrum Z und ii) dem Aussendurchmesser az des distalen Bereichs 10 des Gehäuses 4 im Bereich des Zentrums Z des im Wesentlichen kalottenförmigen distalen Gehäuseendes bzw. des distalen Gehäuseendes in Form der im Wesentlichen zumindest einseitig abgeflachten Kugel kleiner als 1.5. In dem in Figur 5a gezeigten Fall befindet sich das distale Ende 27 der Kanüle 25, und folglich das distale Ende 17 des in der Kanüle 25 gelagerten Lichtleiters 16, im Zentrum Z. In dem in Figur 5b gezeigten Fall ist das distale Ende 27 der Kanüle 25, und folglich das distale Ende 17 des in der Kanüle gelagerten Lichtleiters 16, um einen Abstand S vom Zentrum Z entfernt. Im konkreten Beispiel beträgt dieser Abstand S etwa 0.5 mm, wobei natürlich auch andere Abstände denkbar sind. Diese Ausgestaltungen beziehungsweise Anordnungen in Kombination mit dem homogen streuenden Material des Eindellers 2 führt dazu, dass Lichtreflexe aufgeweitet werden und sich die Leuchtdichte verringert. Dadurch können Lichtreflexe, die den Anwender während der Nutzung stören, vermieden werden. Die störenden Reflexe entstehen in dem Bereich des Sklera-Depressors 2, in welchem sich das distale Ende 27 der Kanüle 25 befindet, also im ersten Bereich bzw. dem distalen Bereich 10. Das aus dem Lichtinstrument 3 transmittierende Licht wird an der inneren Grenzfläche des Gehäuses 4 reflektiert und gelangt zum Auge des Anwenders. Da das distale Ende 27 der Kanüle 25 beim erfindungsgemässen Sklera-Depressor 2 nicht beim distalen Gehäuseende 6 liegt, sondern im Zentrum Z bzw. in der Nähe des Zentrums Z des ersten Bereichs bzw. des distalen Bereichs 10, ist dieser Teil des Sklera-Depressors 2 nicht ständig komplett vom Augen-Gewebe umgeben und das Licht wird folglich nicht oder nur teilweise vom Gewebe absorbiert.

Ein Verlust der Lichtintensität wird weiter verhindert durch die Wahl der Wanddicken dl1, dq1, des Sklera-Depressors 2 in den Bereichen, in welchen das Licht durch das Gehäuse 4 des Sklera-Depessors 2 transmittieren muss. So ist es bevorzugt, den distalen Bereich 10 des Gehäuses 4 mit einer geringen Materialstärke zu bilden, wodurch Lichtverluste aufgrund von Absorption des Materials vermindert werden. Beispielsweise kann das Gehäuse 4 im Bereich 10 des distalen Gehäuseendes 6 bezüglich einer senkrecht zur Längsrichtung L verlaufenden Querrichtung Q eine Wanddicke dq1 von etwa 0.5 mm bis 3 mm, insbesondere von etwa 1.5 mm haben, und/oder das Gehäuse 4 kann im Bereich 10 des distalen Gehäuseendes 6 entlang der Längsrichtung L gesehen eine Wanddicke dl1 von etwa 0.5 mm bis 3 mm, insbesondere von etwa 1 mm haben.

Wie gut in den Figuren 5 und 6 ersichtlich ist bleibt die Wanddicke dq2, dq3 im zweiten und dritten Bereich 11, 12 entlang der Querrichtung Q gesehen jeweils konstant und beträgt im zweiten Bereich 11 in etwa gleich viel wie im dritten Bereich 13. Die lichte Weite W des Aufnahmeraums 8 allerdings nimmt ausgehend vom proximalen Gehäuseende 5 in Richtung des distalen Gehäuseendes 6 gesehen im Wesentlichen kontinuierlich ab. Ein Grund hierfür liegt in der Fertigungstechnik. So muss der Kern "entformt" werden, wofür ein stetiger Winkel nötig ist. Im Spritzgussverfahren ist bei solch anspruchsvollen Bestandteilen eine gleichbleibende Wandstärke eine beinahe schon zwingende Grundvoraussetzung. Weiter steigt durch den immer grösser werdenden Innendurchmesser, also die lichte Weite W, die Biegesteifigkeit.

Der erfindungsgemässe Sklera-Depressor 2, insbesondere eine Aussenseite 13 im Bereich 10 des distalen Gehäuseendes 6, wird vorzugsweise mit einer Rauheit von etwa 0.2 bis 2.2 Ra gemäss EN ISO 1302, vorzugsweise von etwa 0.4 bis 2 Ra gemäss EN ISO 1302, insbesondere bevorzugt von etwa 0.6 bis 0.8 Ra gemäss EN ISO 1302 bereitgestellt. Diese Rauheit wird insbesondere durch den Herstellungsprozess bei der Herstellung der Vorrichtung im Spritzgussverfahren durch Fräs- und Erodierprozesse massgeblich beeinflusst. Zusätzlich zu den günstigen Gleiteigenschaften des transluzenten Materials ermöglicht eine solche Oberflächenrauigkeit ein sehr einfaches Bewegen des Sklera-Depressors 2 auf dem Bulbus (Augapfel), ohne dass das Augengewebe am Sklera-Depressor 2 anhaftet.

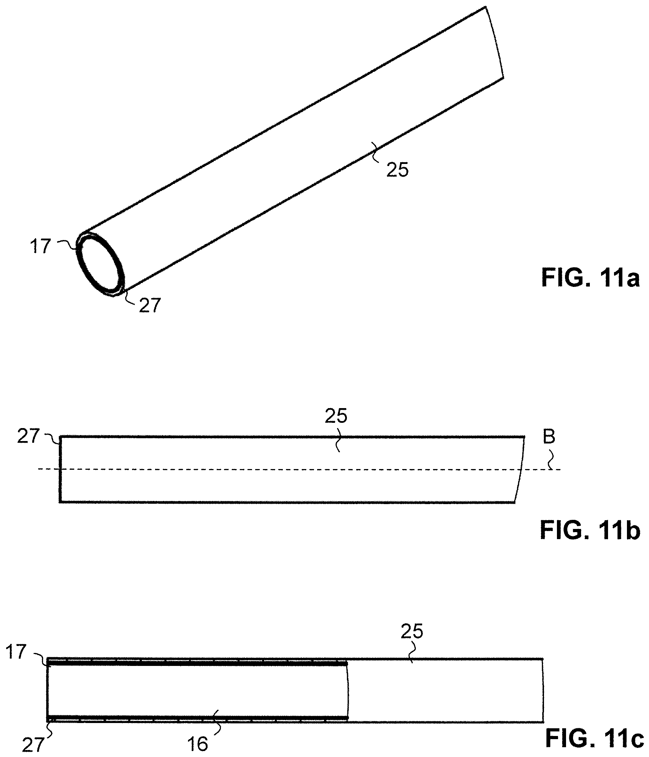

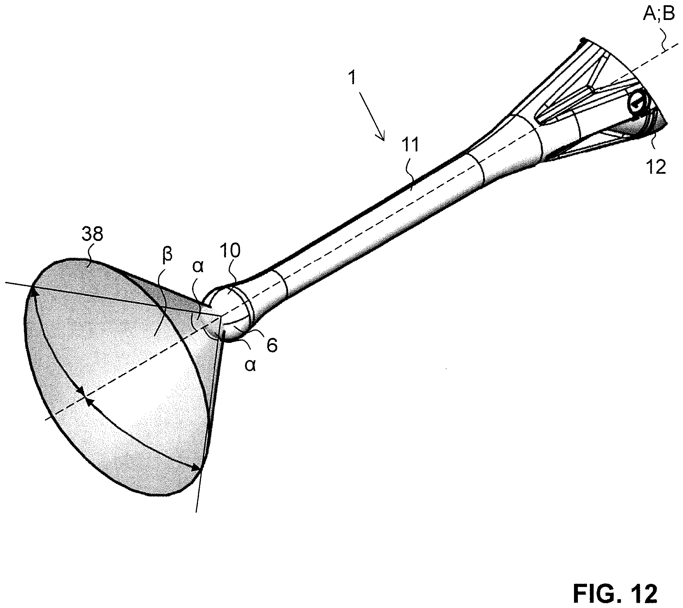

Im Zusammenhang mit den Figuren 10a bis 10d und 11a bis 11c werden verschiedene Aspekte des Lichtinstruments 3, insbesondere von dessen Kanüle 25 und dem Lichtleiter 16, diskutiert. Die daraus resultierenden Abstrahlcharakteristika werden in Bezug auf die Figuren 12 bis 16b erläutert. Wie durch einen Vergleich der Figuren 10a bis 10c und 11a bis 11d hervorgeht, unterscheiden sich die Kanüle 25 und der darin gelagerte Lichtleiter 16 durch dessen distalen Enden 17 und 27. Nämlich, die Kanüle 25 und der Lichtleiter 16 gemäss den Figuren 10a bis 10d weisen jeweils einen konisch geschliffenen Lichtleiter 16 und eine schräg angeschliffene Kanüle 25, was im Gegensatz zum plan geschliffenen Lichtleiter 16 und der plan angeschliffenen Kanüle 25 gemäss den Figuren 11a bis 11c steht. Lichtinstrumente 3 umfassend eine Kanüle 25 und ein Lichtleiter 16 mit jeweils plan geschliffenen distalen Enden 27, 17 werden als "90° Lichtinstrumente" bezeichnet, während Lichtinstrumente 3 umfassend eine Kanüle 25 und Lichtleiter 16 mit schräg angeschliffenem bzw. konisch geschliffenem distalen Ende 27, 17 werden als "Weitwinkel-Lichtinstrumente" bezeichnet. Der schräge Anschliff der Kanüle 25 im Falle des Weitwinkel-Lichtinstruments führt dazu, dass das distale Ende 27 der Kanüle 25 bezüglich einer zentral durch die Kanüle 25 verlaufenden zentralen Längsachse B über einen verkürzten und über einen verlängerten Kanülenbereich verfügt. Der verlängerte Bereich kann auch als abgeschirmter Bereich bezeichnet werden, welcher im Falle von regulären Vorrichtungen aus dem Stand der Technik zum Anwender gerichtet ist um diesen vor einer Blendung des Lichts zu schützen.

Die geometrische Ausgestaltung des distalen Bereichs 10 des Gehäuses 4 zusammen mit dem transluzenten Material, aus welchem der distale Bereich 10 des Gehäuses 4 gebildet ist, bewirken, dass der distale Bereich 10 des Gehäuses 4 das Licht annähernd wie einen diffusen Kugelstrahler abstrahlt. Dies wiederum führt dazu, dass eine gleichmässige Ausleuchtung des eingedellten Augengewebes 34 erreicht wird, und zwar unabhängig vom verwendeten Lichtinstrument 3. Dies soll nun anhand von Messbeispielen gezeigt werden.

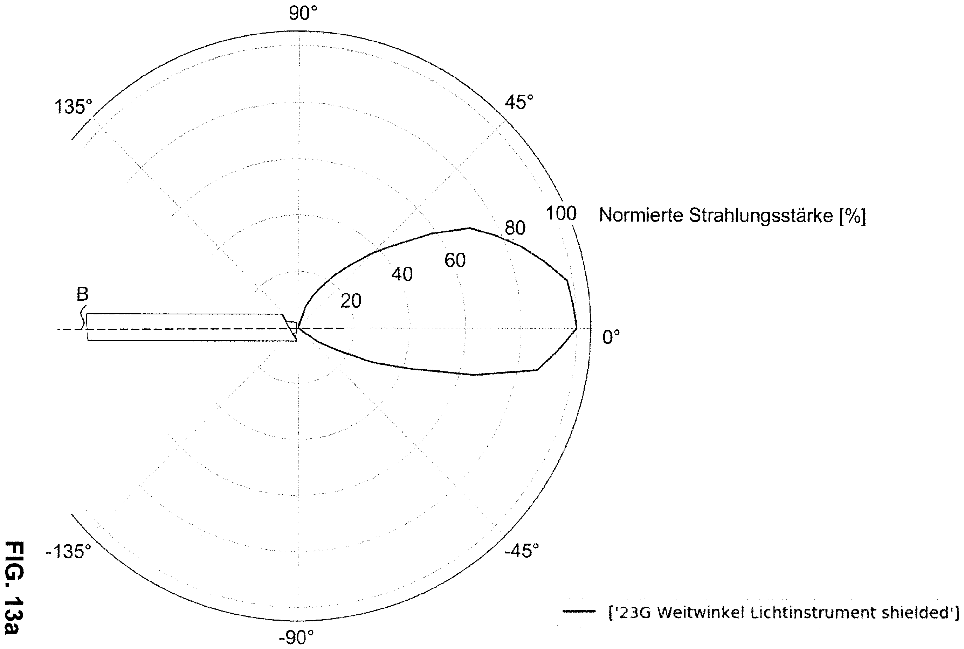

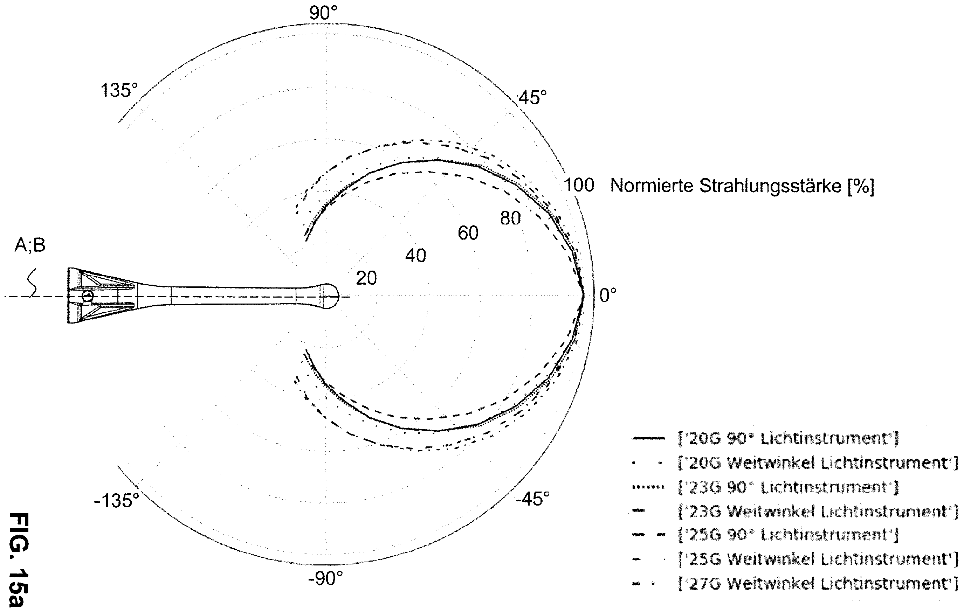

Insbesondere zeigen die Messbeispiele jeweils die Abstrahlcharakteristik von 90° Lichtinstrumenten 3 sowie von Weitwinkel-Lichtinstrumenten 3 mit und ohne Verwendung des erfindungsgemässen Sklera-Depressors 2. Die Abstrahlcharakteristik wird jeweils anhand der Öffnungswinkel α eines kanonischen Raumwinkels β verdeutlicht. Das heisst, und wie aus der schematischen Figur 12 hervorgeht, strahlt die Vorrichtung bzw. der Sklera-Depressor 2 durch dessen distales Gehäuseende 6 Licht 38 des darin gelagerten Lichtinstruments 3 ab. Die geometrische Ausgestaltung und die Beschaffenheit des distalen Gehäuseendes 6 sind dabei derart, dass das Gehäuse 4 im Bereich des distalen Gehäuseendes 6 einen kanonischen Raumwinkel β definiert, welcher 2·π Steradiant beträgt, wobei dieser Raumwinkel β einen Mantel eines geraden Kreiskegels mit einem halben, ebenen Öffnungswinkel α von 90 Grad bildet. Die in den Figuren gezeigten Messungen zeigen jeweils die Strahlungsstärke für verschiedene Öffnungswinkel α bezüglich der zentralen Längsachse B durch das Lichtinstrument 3 (Figuren 13a bis 14) respektive bezüglich der zentralen Längsachse A durch die Vorrichtung bzw. den Sklera-Depressor 2 (Figuren 15a bis 16b).

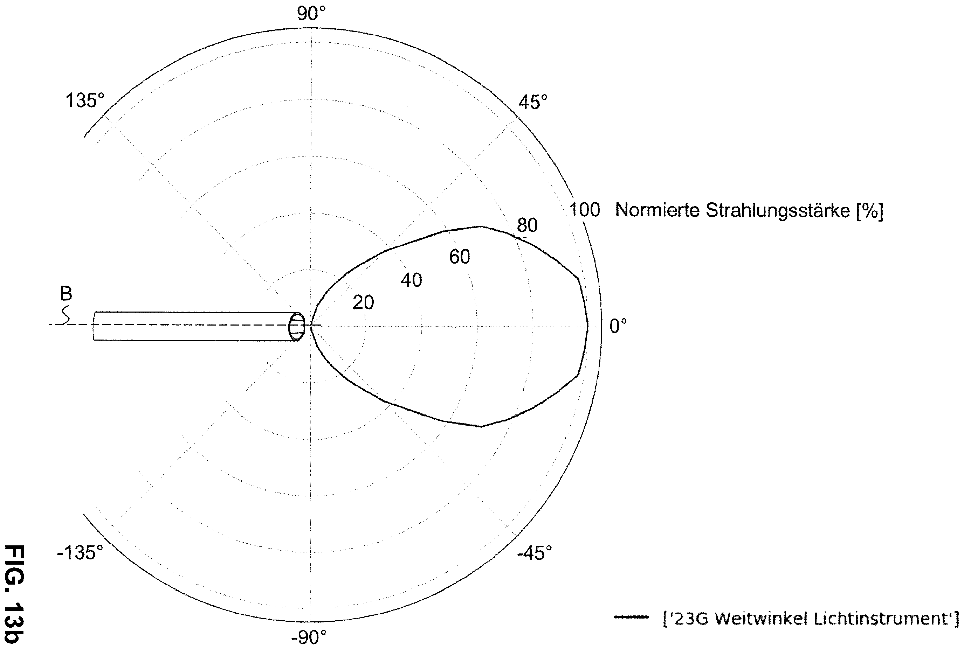

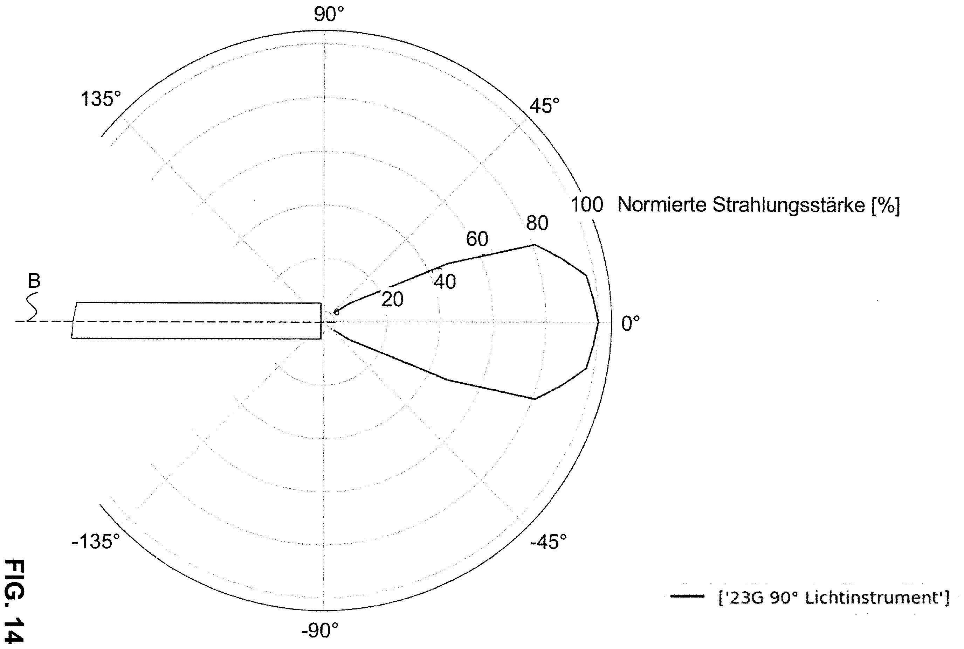

Wie aus Figur 14 hervorgeht, verfügt ein 90° Lichtinstrument über eine symmetrische Strahlungsstärke bezüglich dessen zentrale Längsachse B. Dasselbe gilt auch für das Weitwinkel-Lichtinstrument, wenn die Lichtleistungs-Messung am verkürzten Bereich 36 der Kanüle erfolgt, siehe Figur 13b. Im Falle einer Lichtleistungs-Messung am verlängerten bzw. abgeschirmten Bereich der Kanüle 37 verhält sich die Lichtleistung aufgrund des die Abschirmung bildenden Bereichs der Kanüle asymmetrisch, siehe Figur 13a. Wie nun aus einem Vergleich der Figuren 13a bis 14 mit den Figur 15a bis 16b hervorgeht, ist der erfindungsgemässe Sklera-Depressor 2 in der Lage, die Asymmetrie der Lichtleistung für ein Lichtinstrument 3 umfassend eine Kanüle 25 und Lichtleiter 16 mit schräg angeschliffenem bzw. konisch geschliffenem distalen Ende 27, 17 auszugleichen. Der erfindungsgemässe Sklera-Depressor führt gewissermassen also zu einer Lichtstrahl-Aufweitung.

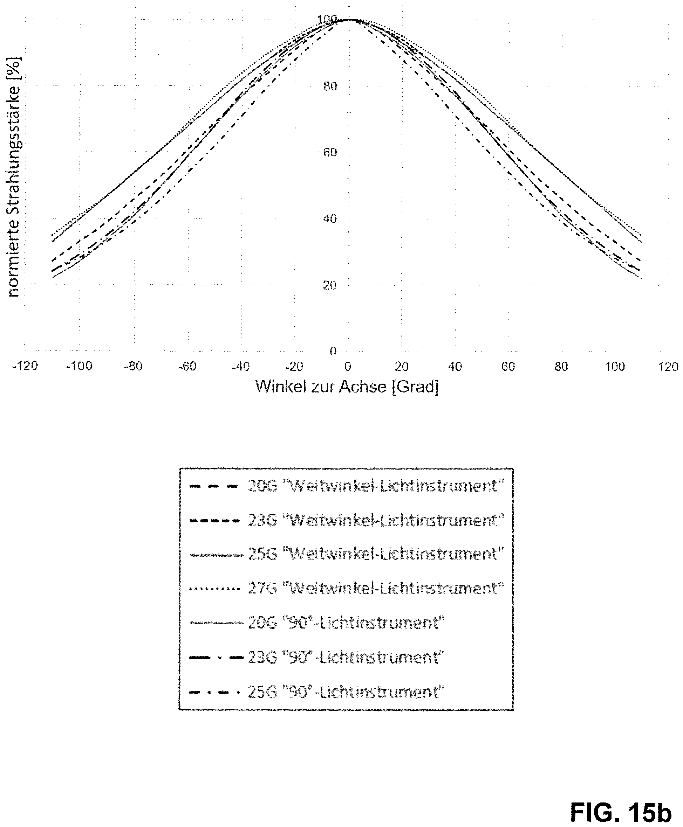

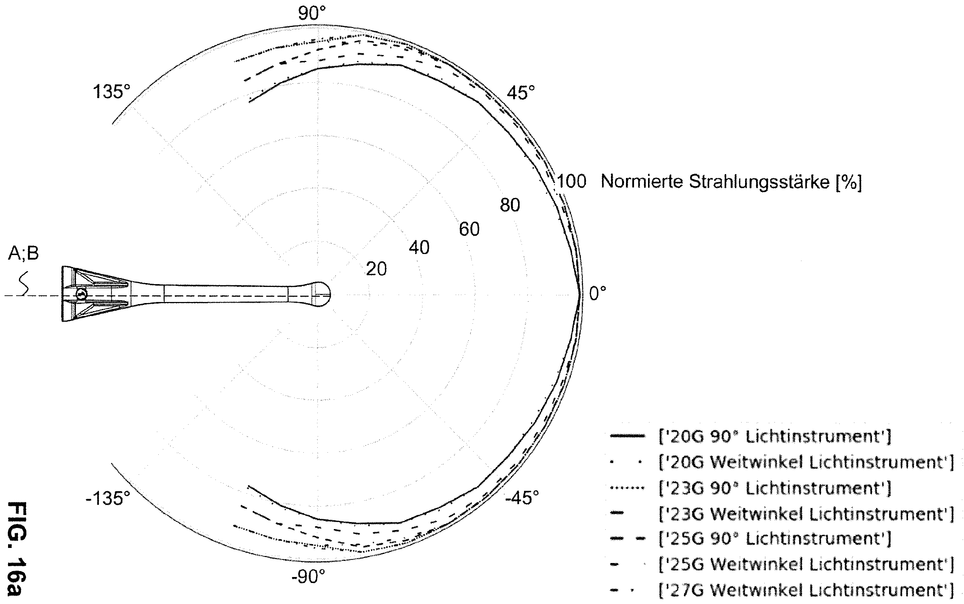

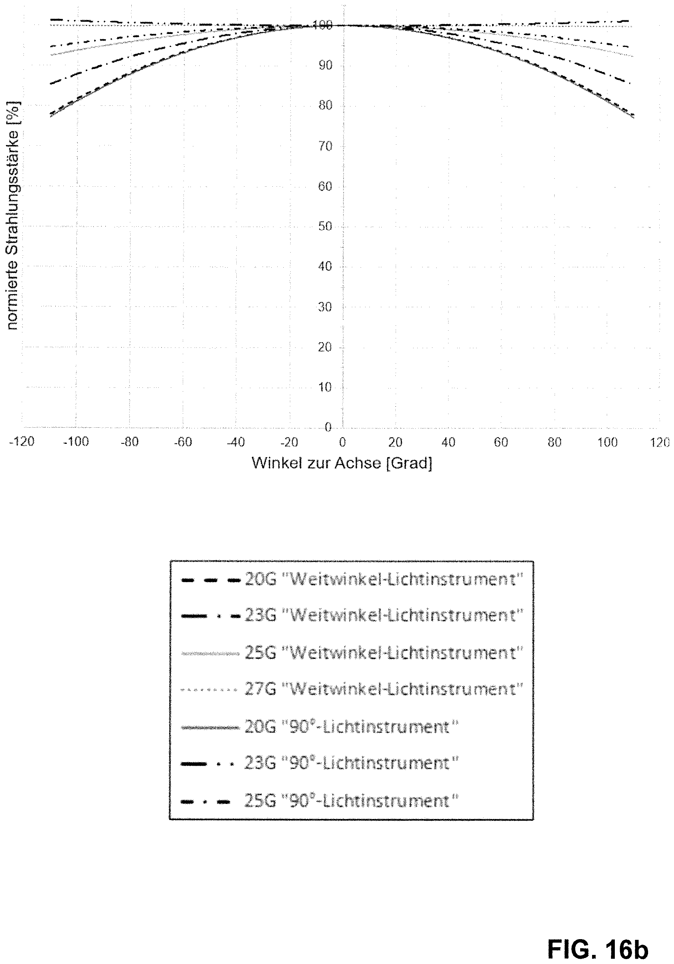

Das heisst, und wie aus den Figuren 15a bis 16b hervorgeht, die Abstrahlcharakteristik des erfindungsgemässen Sklera-Depressors 2 ist derart, dass eine Abweichung der Lichtleistung zwischen dem sogenannten 90° Lichtinstrument und dem sogenannten Weitwinkel-Lichtinstrument für verschiedene Lichtinstrument-Typen etwa 20% oder weniger beträgt. Wie aus den Legenden in den Figuren 15a bis 16b hervorgeht, handelt es sich bei diesen verschiedenen Lichtinstrument-Typen um die dem Fachmann bekannten 20G, 23G, 25G, und 27G Typen, wobei "G" für Gauge steht. Die Vorrichtung 2 bzw. der Sklera-Depressor, welcher für die Messungen der Figuren 15a bis 16b verwendet wurden unterscheidet sich jeweils in seiner Wanddicke. Das heisst, der Sklera-Depressor gemäss den Figuren 15a und 15b umfasst ein Gehäuse 4, welches im Bereich des distalen Gehäuseendes entlang der Längsrichtung L gesehen eine Wanddicke dl1 von etwa 0.8 Millimeter, während die Wanddicke dl1 des Sklera-Depressors gemäss den Figuren 16a und 16b in etwa 2.2 Millimeter beträgt. In beiden Fällen weist der Sklera-Depressor einen kalottenförmigen distalen Endbereich auf. Wie zudem aus den Figuren 15a bis 16b hervorgeht, wird mit dem erfindungsgemässen Sklera-Depressor 2 weiter eine Strahlungsstärke über einen grossen Winkelbereich erreicht. Insbesondere erlaubt der erfindungsgemässe Sklera-Depressor 2 ein Abstrahlen von Licht durch dessen distalen Bereich 10 des Gehäuses 4 mit einer Strahlungsstärke von etwa 20 % oder mehr für den Sklera-Depressor gemäss Figuren 15a und 15b (Wanddicke dl1 von etwa 0.8 Millimeter) bzw. von etwa 80% oder mehr für den Sklera-Depressor 2 gemäss Figuren 16a und 16b (Wanddicke dl1 von etwa 2.2 Millimeter) für einen Öffnungswinkel α von grösser als 100°. Dadurch, dass der Sklera-Depressor 2 über eine sehr gleichmässige Abstrahlcharakteristik verfügt, besteht der weitere Vorteil, dass die zulässige Behandlungszeit erheblich erhöht werden kann ohne dass phototoxische Reaktionen im Gewebe des Auges entstehen.

**BEZUGSZEICHENLISTE**

| | | | |
|---|---|---|---|
| 1 | ophthalmologisches | 31 | Vorsprünge |
| | Beleuchtungssystem | 32 | Aussenseite |
| 2 | Vorrichtung | 33 | Auge |
| 3 | Lichtinstrument | 34 | Augengewebe |
| 4 | Gehäuse | 35 | Markierung |
| 5 | proximales Gehäuseende | 36 | verkürzter Bereich |
| 6 | distales Gehäuseende | 37 | verlängerter Bereich |
| 7 | Öffnung | 38 | Licht |
| 8 | Aufnahmeraum | 39 | Rippe |
| 9 | Sklera | 40 | Aussendurchmesser |
| 10 | erster Bereich | | |
| 11 | zweiter Bereich | | |
| 12 | dritter Bereich | A | zentrale Längsachse der |
| 13 | Aussenseite | | Vorrichtung |
| 14 | Innenseite | B | zentrale Längsachse des |
| 15 | Verstärkungsrippe | | Lichtinstruments |
| 16 | Lichtleiter | S | Abstand |
| 17 | distales Ende | L | Längsrichtung |
| 18 | proximales Ende | T | Trennrichtung |
| 19 | Okkular | V | Verbindungsrichtung |
| 20 | Operationsmikroskop | Q | Querrichtung |
| 21 | Strahlengang | w | lichte Weite |
| 22 | Vitrektom | Z | Zentrum |
| 23 | Trokar | dl1 | Wanddicke |
| 24 | Handgriff | dq1 | Wanddicke |
| 25 | _{Kanüle} | dq2 | Wanddicke |
| 26 | Stecker | dq3 | Wanddicke |
| 27 | distales Ende | a1 | Aussendurchmesser |
| 28 | distaler Bereich | a2 | Aussendurchmesser |
| 29 | distales Ende | a3 | Aussendurchmesser |
| 30 | Zwischenraum | az | Aussendurchmesser |
| α | Öffnungswinkel | β | Raumwinkel |

## Patentansprüche

1. Vorrichtung (2) für ein ophthalmologisches Beleuchtungssystem (1) umfassend ein Lichtinstrument (3) zur Beleuchtung des intraokularen Raumes eines menschlichen oder tierischen Auges (33),
wobei die Vorrichtung (2) ein Gehäuse (4) mit einem proximalen Gehäuseende (5), einem distalen Gehäuseende (6), und eine Öffnung (7) im proximalen Gehäuseende (5) umfasst,
wobei das Gehäuse (4) einen Aufnahmeraum (8) begrenzt, welcher sich ausgehend von der Öffnung (7) im proximalen Gehäuseende (5) entlang einer Längsrichtung (L) in Richtung des distalen Gehäuseendes (6) erstreckt, und
wobei der Aufnahmeraum (8) zur Aufnahme des Lichtinstruments (3) durch die Öffnung (7) im proximalen Gehäuseende (5) ausgebildet ist, **dadurch gekennzeichnet, dass** das Gehäuse (4) zumindest im Bereich (10) des distalen Gehäuseendes (6) mindestens ein transluzentes Material umfasst.

2. Vorrichtung (2) gemäss Anspruch 1, wobei das transluzente Material ein transluzenter Kunststoff, insbesondere ein transluzenter technischer Kunststoff, bevorzugt ein transluzenter teilkristalliner Kunststoff, am bevorzugtesten ein Polyoxymethylen Copolymer ist, und/oder
wobei das transluzente Material ein Kunststoff und/oder ein Silikon mit Partikeln ist, wobei die Partikel dazu ausgebildet sind, auftreffendes Licht, welches vom Lichtinstrument (3) emittiert wird, zu streuen, und/oder
wobei das transluzente Material eine Absorptionskonstante (k) von etwa 10^(-3) im sichtbaren Wellenlängenbereich aufweist.

3. Vorrichtung (2) gemäss einem der vorhergehenden Ansprüche, wobei die Vorrichtung (2) zur Indentation des Augengewebes (34), insbesondere der Sklera (9), ausgebildet ist.

4. Vorrichtung (2) gemäss einem der vorhergehenden Ansprüche, wobei das Gehäuse (4) mindestens einen ersten Bereich (10) und einen sich daran anschliessenden zweiten Bereich (11) umfasst, wobei das distale Gehäuseende (6) im ersten Bereich (10) angeordnet ist, wobei ein Aussendurchmesser (a1) des ersten Bereichs (10) grösser als ein Aussendurchmesser (a2) des zweiten Bereichs ist (11), und
wobei ein Verhältnis zwischen dem Aussendurchmesser (a1) des ersten Bereichs (10) und dem Aussendurchmesser (a2) des zweiten Bereichs (11) insbesondere mehr als 1 beträgt, und/oder
wobei das Verhältnis zwischen dem Aussendurchmesser (a1) des ersten Bereichs (10) und dem Aussendurchmesser (a2) des zweiten Bereichs (11) insbesondere zwischen 1.1 und 2.0, vorzugsweise zwischen 1.3 und 1.7, und insbesondere bevorzugt etwa 1.5 beträgt.

5. Vorrichtung (2) gemäss einem der vorhergehenden Ansprüche, wobei das distale Gehäuseende (6) im Wesentlichen kalottenförmig ausgebildet ist oder die Form einer im Wesentlichen zumindestens einseitig abgeflachten Kugel aufweist, und/oder
wobei das distale Gehäuseende (6) bezüglich einer zentralen Längsachse (A) des Gehäuses (4) einen Öffnungswinkel (α) für austretendes Licht des Lichtinstruments (3) von grösser als 100°, vorzugsweise von etwa 110° gemäss EN-ISO 15752:2010 definiert, und/oder
wobei das Gehäuse (4) im Bereich des distalen Gehäuseendes (6) einen kanonischen Raumwinkel (β) definiert, welcher 2·π Steradiant beträgt, wobei dieser Raumwinkel (β) einen Mantel eines geraden Kreiskegels mit einem halben, ebenen Öffnungswinkel (α) von 90 Grad bildet, so dass innerhalb dieses Raumwinkels (β) eine Strahlungsstärke des aus dem Bereich des distalen Gehäuseendes (6) austretenden Lichts (38) des Lichtinstruments (3) mindestens 30 %, vorzugsweise mindestens 60 %, insbesondere bevorzugt mindestens 90 % einer Strahlungsstärke des Lichts (38) des Lichtinstruments (3) innerhalb dieses Raumwinkels (β) beträgt.

6. Vorrichtung (2) gemäss einem der vorhergehenden Ansprüche, wobei das Gehäuse (4) einen dritten Bereich (12) umfasst, wobei das proximale Gehäuseende (5) im dritten Bereich (12) angeordnet ist, und wobei der dritte Bereich (12) ausgehend vom proximalen Gehäuseende (5) in Richtung des distalen Gehäuseendes (6) gesehen zumindest teilweise in Richtung der Längsrichtung (L) nach innen zulaufend ausgebildet ist.

7. Vorrichtung (2) gemäss einem der vorhergehenden Ansprüche, wobei eine lichte Weite (W) des Aufnahmeraums (8) ausgehend vom proximalen Gehäuseende (5) in Richtung des distalen Gehäuseendes (6) gesehen vorzugsweise im Wesentlichen kontinuierlich abnimmt.

8. Vorrichtung (2) gemäss einem der vorhergehenden Ansprüche, wobei das Gehäuse (4) im Bereich des distalen Gehäuseendes (6) bezüglich einer senkrecht zur Längsrichtung (L) verlaufenden Querrichtung (Q) eine Wanddicke (dq1) von etwa 0.5 mm bis 3 mm, insbesondere von etwa 1.5 mm hat, und/oder
wobei das Gehäuse (4) im Bereich des distalen Gehäuseendes (6) entlang der Längsrichtung (L) gesehen eine Wanddicke (dl1) von etwa 0.5 mm bis 3 mm, insbesondere von etwa 1 mm hat.

9. Vorrichtung (2) gemäss einem der vorhergehenden Ansprüche, wobei das Gehäuse (4) auf einer Aussenseite (13) zumindest im Bereich des distalen Gehäuseendes (6) eine Rauheit von etwa 0.2 bis 2.2 Ra gemäss EN ISO 1302, vorzugsweise von etwa 0.4 bis 2 Ra gemäss EN ISO 1302, insbesondere bevorzugt von etwa 0.6 bis 0.8 Ra gemäss EN ISO 1302 aufweist.

10. Vorrichtung (2) gemäss einem der vorhergehenden Ansprüche, wobei die Vorrichtung (2) mit dem Lichtinstrument (3) lösbar verbindbar ist, und wobei das Gehäuse (4) insbesondere zur Ausbildung einer formschlüssigen und/oder kraftschlüssigen Verbindung mit dem Lichtinstrument (3) ausgebildet ist.

11. Vorrichtung (2) gemäss einem der vorhergehenden Ansprüche, wobei die Vorrichtung (2) mit Ausnahme der Öffnung (7) im proximalen Gehäuseende (5) vollständig geschlossen ausgebildet ist.

12. Vorrichtung (2) gemäss einem der vorhergehenden Ansprüche, wobei das Gehäuse (4) im Bereich des proximalen Gehäuseendes (5) auf einer dem Aufnahmeraum (8) zugewandten Innenseite (14) über mindestens eine Verstärkungsrippe (15) verfügt.

13. Ophthalmologisches Beleuchtungssystem (1) umfassend eine Vorrichtung (2) gemäss einem der vorhergehenden Ansprüche und ein Lichtinstrument (3), wobei das Lichtinstrument (3) vorzugsweise einen Lichtleiter (16) zum Leiten von Licht umfasst.

14. Ophthalmologisches Beleuchtungssystem (1) gemäss Anspruch 13, wobei der Lichtleiter (16) derart im Aufnahmeraum (8) der Vorrichtung (2) gelagert ist, dass ein Verhältnis zwischen
i. einem Abstand (S) zwischen einem distalen Ende (17) des Lichtleiters (16) und einem Zentrum (Z) des distalen Gehäuseendes (6), insbesondere dem Zentrum (Z) des im Wesentlichen kalottenförmigen distalen Gehäuseendes (6) oder des distalen Gehäuseendes (6) in Form der im Wesentlichen zumindest einseitig abgeflachten Kugel, und
ii. dem Aussendurchmesser (a1) des ersten Bereichs (10) des Gehäuses (4), insbesondere der Aussendurchmesser (az) im Bereich des Zentrums (Z) des im Wesentlichen kalottenförmigen distalen Gehäuseendes (6) oder des distalen Gehäuseendes (6) in Form der im Wesentlichen zumindest einseitig abgeflachten Kugel
kleiner als 1.5 ist, und/oder
wobei das distale Ende (17) des Lichtleiters (16) im Zentrum (Z) des im Wesentlichen kalottenförmigen distalen Gehäuseendes (6) oder des distalen Gehäuseendes (6) in Form der im Wesentlichen zumindest einseitig abgeflachten Kugel angeordnet ist.

15. Verfahren zur Herstellung einer Vorrichtung für ein ophthalmologisches Beleuchtungssystem (1) umfassend ein Lichtinstrument (3), insbesondere einer Vorrichtung (2) gemäss einem der vorhergehenden Ansprüche 1 bis 12, zur Beleuchtung des intraokularen Raumes eines menschlichen oder tierischen Auges (33) umfassend den Schritt von:
Spritzgiessen eines Gehäuses (4) mit einem proximalen Gehäuseende (5), einem distalen Gehäuseende (6), und einer Öffnung (7) im proximalen Gehäuseende (5) unter Verwendung eines Spritzgusswerkzeuges, wobei ein Aufnahmeraum (8) im Gehäuse (4) gebildet wird, welcher sich ausgehend von der Öffnung (7) im proximalen Gehäuseende (5) entlang einer Längsrichtung (L) in Richtung des distalen Gehäuseendes (6) erstreckt, und wobei zumindest für den Bereich des distalen Gehäuseendes (6) mindestens ein transluzentes Material verwendet wird.
